# EUROPEAN PATENT APPLICATION

(11) **EP 2 692 362 A1**
(43) Date of publication of application: **05.02.2014**
(21) Application number: 12178893.9
(22) Date of filing: 01.08.2012
(51) Int. Cl.: A61L 2/26, A23L 3/3409, B65D 81/28

(54) **Packaging having anti-mold coating**

(71) Applicant: Yiu, Wen-Shi, Taichung City 429 (TW)
(72) Inventor: Yiu, Wen-Shi, Shengang Dist., Taichung City 429 (TW)
(74) Representative: 2K Patentanwälte Blasberg Kewitz & Reichel

(57) **Abstract**

A durable anti-mold packet comprises a holding pouch (10), an absorption material (25) and an anti-mold evaporation substance (20). The holding pouch (10) has an upper edge (12), a lower edge (13), a left edge (14) and a right edge (15). The left edge (14) and right edge (15) are folded and overlapped to form a housing space (16) and an overlapped seam edge (17). The upper edge (12) and lower edge (13) are pressed and sealed. The overlapped seam edge (17) is partially pressed and sealed to form an evaporation vent (18) communicating with the exterior. The anti-mold evaporation substance (20) is coated onto the absorption material (25) which is held in the housing space (16). Therefore a greater amount of the anti-mold evaporation substance (20) can be absorbed by the absorption material (25) and the anti-mold evaporation substance (20) can be slowly released from the absorption material (25) to increase use duration of the anti-mold packet and provide anti-mold effect for a longer period to meet use requirements.

## Description

### FIELD OF THE INVENTION

The present invention relates to an anti-mold article and particularly to a packet to hold an anti-mold evaporation substance.

### BACKGROUND OF THE INVENTION

In damp and warm environments, such as Southeast Asia regions, plum rain seasons or the like, how to prevent foods or leather goods from becoming moldy in a long period is an issue bothering many people. One of the conventional techniques is to dispose chemicals such as antiseptic, desiccant or deoxidizer in a container to prohibit or prevent mold from breeding. But in an undesirable environment, such as a prolonged rainy season, if the efficacy period of the chemicals is short and their replacement is not being done regularly, moldy problem could still occur. Moreover, the chemicals with a longer efficacy period often could be harmful to people's health. Used on foods the chemicals could produce ill effects on the health of human being, thus there are still rooms for improvement.

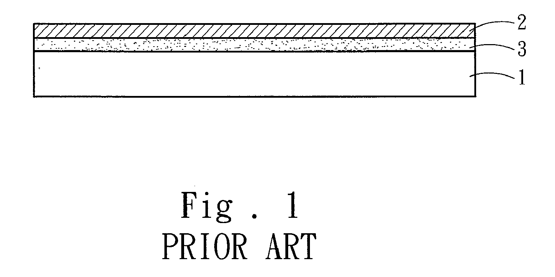

At present, there are patches and tags absorbed with anti-mold essence on the market. Please refer to FIG. 1 for one of such goods. It generally is made in a sheet structure including a base sheet 1, an upper sheet 2 and an essence absorption layer 3 sandwiched between the base sheet 1 and upper sheet 2. The essence absorption layer 3 absorbs an anti-mold essence and can release the essence from the edge in contact with the air. The base sheet 1 and upper sheet 2 also are capable of releasing the essence depending on their structure and material. However, the essence absorption layer 3 made in the sheet structure cannot contain too much essence. After used for a period of time, the anti-mold essence is exhausted through evaporation, and replacement is needed. Hence its usability is lower and cannot meet the requirement of longer use period.

Another conventional technique is disclosed in R.O.C. patent No. M290754 which includes a release paper and a paster bonded together through adhesive. The paster contains an anti-mold composition capable of volatilizing. When the release paper is peeled off, the paster can be bonded into a container with goods held inside. The anti-mold composition is released continuously for a preset period of time to prevent the goods from becoming moldy. The anti-mold composition consists of 30% of garlic oil, 5% of tea tannin, 40% of capsicum oil, 20% of mustard powder and 5% of citronella.

The garlic oil includes ingredients of sulfide, propylene sulfur, propylene cysteire, sulfur oxide, propylene isothiocyanate and the like that can provide deodorization and antibacterial effect. The tea tannin is generally called as polyphenol extracted from tea that also contains flavanols which can provide deodorization, antibacterial, antiseptic, anti-mold and antioxidation effect. The capsicum oil contains aromatic hydrocarbon which is antibacterial and can be volatilized to produce vapor to form a protective film to shield insects and also provide preservation effect. The mustard powder has a strong disinfection effect. The citronella mainly consists of 70-80% of citral and 20% of myrcene, and also includes other ingredients such as citronellal (C10H180), geraniol (C10H180), 1-borneol, 1,8-p-menthadien-5-ol and the like that also provide antibacterial and deodorization effect.

Bottle-contained anti-mold essence can last longer in use, but is prone to spill out to cause contamination. It also leaves a lot to be desired in terms of usability.

### SUMMARY OF THE INVENTION

The primary object of the present invention is to provide a durable anti-mold packet to overcome the problem of the conventional techniques that contain a small amount of anti-mold essence so that the anti-mold essence has to be replaced frequently due to fast evaporation.

To achieve the foregoing object, the durable anti-mold packet provided by the invention comprises a holding pouch, an absorption material and an anti-mold evaporation substance. The holding pouch has an upper edge, a lower edge, a left edge and a right edge. The left edge and right edge are folded and overlapped to form a housing space and an overlapped seam edge. The upper edge and lower edge are pressed and sealed. The overlapped seam edge is partially pressed and sealed to form an evaporation vent communicating with the exterior. The anti-mold evaporation substance is coated onto the absorption material which is held in the housing space.

By means of the structure set forth above, a greater amount of the anti-mold evaporation substance can be absorbed by the absorption material so that the anti-mold evaporation substance can be released slowly. The holding pouch also can hold the anti-mold evaporation substance to further increase the amount of the anti-mold evaporation substance therein. The evaporation vent formed on the overlapped seam edge can be controlled at a desired size to limit evaporation amount of the anti-mold evaporation substance to apply to different use environments. Thus through control of the absorption of the absorption material and the size of the evaporation vent, the release duration of the anti-mold evaporation substance can be increased to provide a durable anti-mold packet usable for a prolonged period of time to meet user's requirements.

The foregoing, as well as additional objects, features and advantages of the invention will be more readily apparent from the following detailed description, which proceeds with reference to the accompanying drawings.

### BRIEF DESCRIPTION OF THE DRAWINGS

FIG. 1 is a cross section of a conventional technique.
FIG. 2 is a schematic view of the invention in a spread condition.
FIG. 3 is a schematic view of the invention in an assembled condition.
FIG. 4 is a schematic view of the invention in a use condition.
FIG. 5 is a schematic view of an embodiment of the invention.

### DETAILED DESCRIPTION OF THE PREFERRED EMBODIMENT

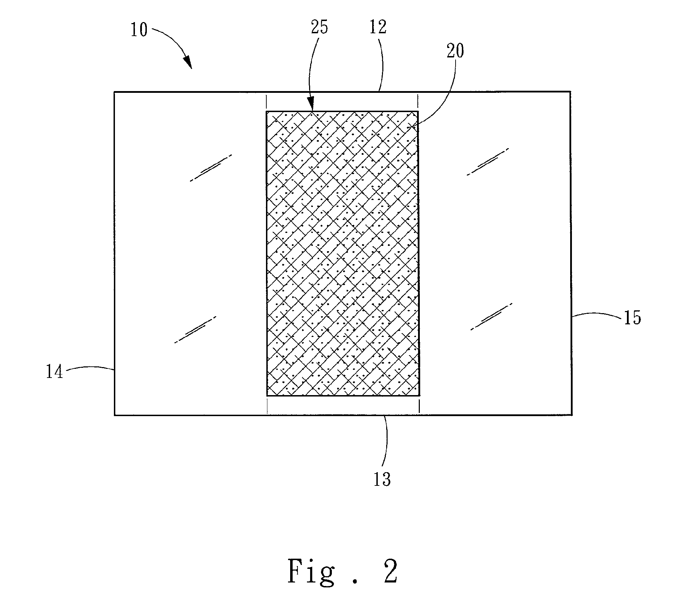

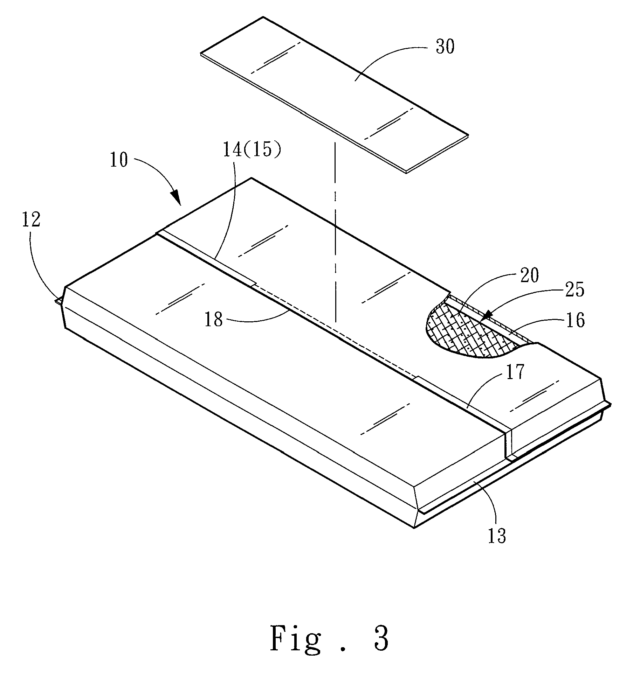

Please refer to FIGS. 2 and 3, the present invention aims to provide a durable anti-mold packet which comprises a holding pouch 10, an absorption material 25 and an anti-mold evaporation substance 20. The holding pouch 10 has an upper edge 12, a lower edge 13, a left edge 14 and a right edge 15. The left edge 14 and right edge 15 are folded and overlapped to form a housing space 16 and an overlapped seam edge 17. The upper edge 12 and lower edge 13 are pressed and sealed. The overlapped seam edge 17 is partially pressed and sealed to form an evaporation vent 18 communicating with the exterior. The evaporation vent 18 can be located at the center of the overlapped seam edge 17. The holding pouch 10 can be made of nylon or other environmental-friendly material, such as aluminum foil or the like.

The anti-mold evaporation substance 20 is coated onto the absorption material 25 which is held in the housing space 16. The absorption material 25 is porous, and can be made of non-woven fabric or other porous material with desirable absorption capability, hence can absorb liquid substance, such as the anti-mold evaporation substance 20 which is substantially formed in a liquid state. After the anti-mold evaporation substance 20 has been absorbed by the absorption material 25, it can be slowly released. Thus not only a greater amount of the anti-mold evaporation substance 20 can be held, the anti-mold effect also can be prolonged. The invention also can include a sealing patch 30 to cover and seal the evaporation vent 18, thus the housing space 16 also can be sealed by the sealing patch 30.

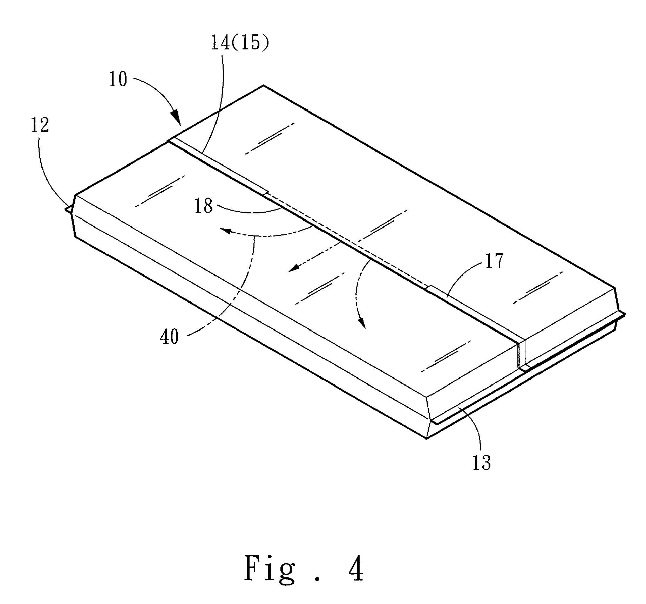

Also referring to FIG. 4, when in use, once the sealing patch 30 is peeled off, the housing space 16 can communicate with the exterior through the evaporation vent 18, hence the anti-mold evaporation substance 20 can become gas 40 and slowly release from the absorption material 25 to achieve anti-mold effect. The porous absorption material 25 can absorb the anti-mold evaporation substance 20. The width of the overlapped seam edge 17 and length of the evaporation vent 18 can be changed as desired. In other words, in response to different requirements, their size can be selected and controlled to change the release speed of the anti-mold evaporation substance 20 to meet requirements in different environments.

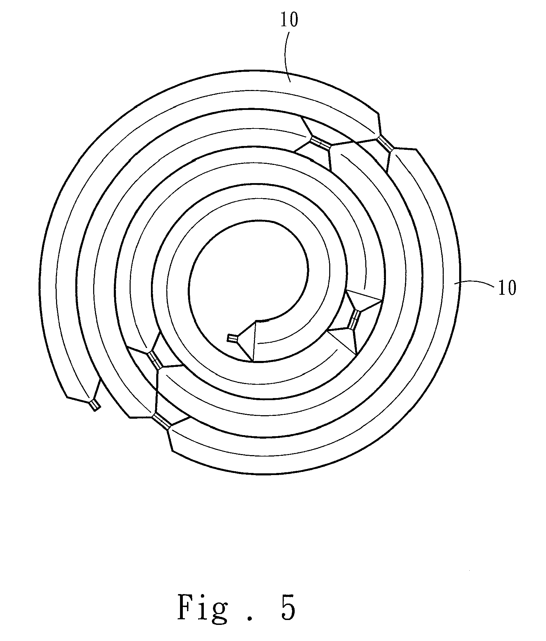

Please refer to FIG. 5, the holding pouch 10 of the invention can also be formed in multiple sets and clustered and wound in a roll film. When in use, each holding pouch 10 can be detached individually to facilitate using and storage.

As a conclusion, compared with the conventional techniques, the invention provides features as follows:
1. Through the porous characteristic of the absorption material, a great amount of the anti-mold evaporation substance can be absorbed by the absorption material so that the anti-mold evaporation substance can be slowly released to extend the use period.
2. By holding the anti-mold evaporation substance in the holding pouch, the amount of the anti-mold evaporation substance therein can be further increased. Hence release duration of the anti-mold evaporation substance can be increased, and the lifespan of the anti-mold packet is longer.
3. Through changing the width of the overlapped seam edge and length of the evaporation vent, the release speed of the anti-mold evaporation substance can be controlled to meet different use requirements at different sites.
4. By holding the anti-mold essence through the holding pouch the anti-mold essence can be prevented from spilling out to avoid the problem of contamination.

## Claims

1. A durable anti-mold packet, **characterized by**:
a holding pouch (10) including an upper edge (12), a lower edge (13), a left edge (14) and a right edge (15), the left edge (14) and the right edge (15) being folded and overlapped to form a housing space (16) and an overlapped seam edge (17), the upper edge (12) and the lower edge (13) being pressed and sealed, the overlapped seam edge (17) being partially pressed and sealed to form an evaporation vent (18) communicating with exterior;
an absorption material (25); and
an anti-mold evaporation substance (20) coated onto the absorption material (25) which is held in the housing space (16).

2. The durable anti-mold packet of claim 1 further including a sealing patch (30) to cover and seal the evaporation vent (18).

3. The durable anti-mold packet of claim 1 or 2, wherein the evaporation vent (18) is located at a center of the overlapped seam edge (17).

4. The durable anti-mold packet of any of the claims 1 to 3, wherein the holding pouch (10) is made of a material selected from the group consisting of nylon and aluminum foil.

5. The durable anti-mold packet of any of the claims 1 to 4, wherein the absorption material (25) is a non-woven fabric.

6. The durable anti-mold packet of any of the claims 1 to 5, wherein the holding pouch (10) includes multiple sets clustered and wound in a roll film.

## Amended claims

### Amended claims in accordance with Rule 137(2) EPC.

**1.** A durable anti-mold packet, comprising:
a holding pouch (10) including an upper edge (12), a lower edge (13), a left edge (14) and a right edge (15), wherein the left edge (14) and the right edge (15) are folded and overlapped to form a housing space (16) and an overlapped seam edge (17);
an anti-mold evaporation substance (20) being formed in a liquid state and evaporated outside the holding pouch (10); and
a porous absorption material (25) being held in the housing space (16) of the holding pouch (10) to absorb and store the anti-mold evaporation substance (20),
**characterized in that** the upper edge (12) and the lower edge (13) of the holding pouch (10) are pressed and sealed, and the overlapped seam edge (17) is partially pressed and sealed to form an evaporation vent (18) communicating with exterior.

**2.** The durable anti-mold packet of claim 1, further including a sealing patch (30) to cover and seal the evaporation vent (18).

**3.** The durable anti-mold packet of claim 1 or 2, wherein the evaporation vent (18) is located at a center of the overlapped seam edge (17).

**4.** The durable anti-mold packet of any of the claims 1 to 3, wherein the holding pouch (10) is made of a material selected from the group consisting of nylon and aluminum foil.

**5.** The durable anti-mold packet of any of the claims 1 to 4, wherein the porous absorption material (25) is a non-woven fabric.

**6.** The durable anti-mold packet of any of the claims 1 to 5, wherein the holding pouch (10) includes multiple sets clustered and wound in a roll film.
